# EUROPEAN PATENT SPECIFICATION

(11) **EP 2 443 094 B1**
(45) Date of publication and mention of the grant of the patent: **20.03.2013**
(21) Application number: 10724533.4
(22) Date of filing: 21.06.2010
(51) Int. Cl.: C07D 235/20, A61K 31/4184, A61P 9/12

(54) **PROCESS FOR THE PREPARATION OF TELMISARTAN**
VERFAHREN ZUR HERSTELLUNG VON TELMISARTAN
PROCÉDÉ POUR LA PRÉPARATION DE TELMISARTAN

(30) Priority: 19.06.2009 SI 200900169; 30.09.2009 SI 200900267; 18.12.2009 SI 200900387
(43) Date of publication of application: 25.04.2012
(73) Proprietor: Krka Tovarna Zdravil, D.D., Novo Mesto, 8501 Novo Mesto (SI)
(72) Inventor: ZUPANCIC, Silvo, 8000 Novo mesto (SI); SEDMAK, Gregor, 1000 Ljubljana (SI); VRECER, Franc, 8351 Straza pri Novem mestu (SI)
(74) Representative: UEXKÜLL & STOLBERG
(86) International application number: PCT/EP2010/058754
(87) International publication number: WO 2010/146187

(56) References cited:
- WO-A1-2004/087676
- WO-A1-2005/108375
- WO-A2-2007/147889
- US-A1- 2006 264 491
- GUO X Z ET AL: "Synthesis and biological activities of novel nonpeptide angiotensin II receptor antagonists based on benzimidazole derivatives bearing a heterocyclic ring", BIOORGANIC & MEDICINAL CHEMISTRY, PERGAMON, GB LNKD- DOI:10.1016/J.BMC.2008.10.040, vol. 16, no. 24, 15 December 2008 (2008-12-15), pages 10301-10310, XP025685121, ISSN: 0968-0896 [retrieved on 2008-10-22]

## Description

The invention relates to a process for preparing telmisartan or its pharmaceutically acceptable salts. Also disclosed is a process for isolating telmisartan or its pharmaceutically acceptable salts. Moreover, a process for preparing a telmisartan cyano intermediate is disclosed. Also disclosed is a multilayer pharmaceutical tablet comprising telmisartan and a diuretic as well as the process for the preparation of a multilayer pharmaceutical tablet.

### Background of the invention

Telmisartan with its chemical name 4'-[(2-n-propyl-4-methyl-6-(1-methylbenzimidazol-2-yl)benzimidazol-1-yl)methyl]biphenyl-2-carboxylic acid and formula 1 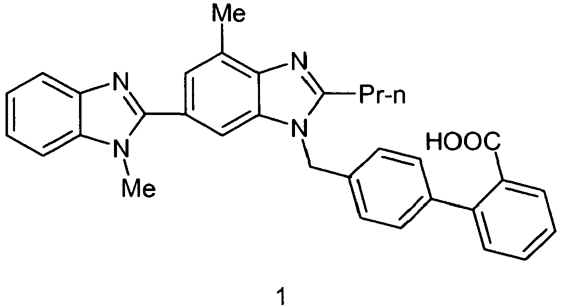 is a non-peptide angiotensin II receptor type AT₁ antagonist useful for the treatment of hypertension. It can be used alone or in combination with another pharmaceutically active compound like a diuretic such as hydrochlorothiazide.

Telmisartan was originally disclosed in EP 0 502 314 A1 and J. Med. Chem. 36 (25), 4040-4051 (1993). Its polymorphs are known from EP 1 144 386 A1, and J. Pharm. Sci, 89 (11), 1465-1479 (2000).

EP 0 502 314 A1 and J. Med. Chem. 36 (25), 4040-4051 (1993) disclose a method for the preparation of telmisartan comprising hydrolysis of the corresponding tert.-butyl ester intermediate into free acid telmisartan. However, WO 2004/087676 reports that such a hydrolysis is difficult to be performed on a large industrial scale. In particular, the final product is said to be difficult to be filtered, washed and isolated.

Therefore, there are various patent applications disclosing the preparation of telmisartan by hydrolysis of other telmisartan intermediates. For example, WO 2004/087676 describes the hydrolysis of a compound with the chemical name 4'-((1,7'-dimethyl-2'-propyl-1H,3'H-2,5'-bibenzo[d]imidazol-3'-yl)-methyl)biphenyl-2-carbonitrile and having formula 2 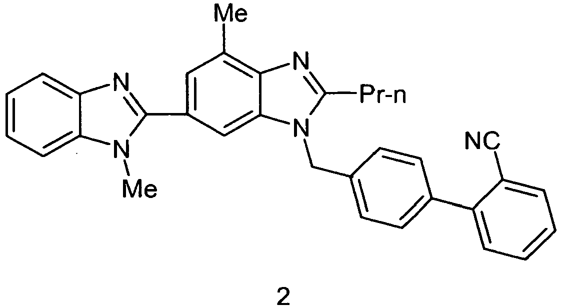 which is hereinafter referred to as cyanotelmisartan. In particular, the hydrolysis of cyanotelmisartan is carried out at elevated temperatures using strong alkaline conditions. Also, CN 1412183 discloses the hydrolysis of cyanotelmisartan.

US 2006/0264491 A1 discloses the hydrolysis of 4'-((1,7'-dimethyl-2'-propyl-1H,3'H-2,5'-bibenzo[d]imidazol-3'-yl)methyl)biphenyl-2-carboxamide having formula 3 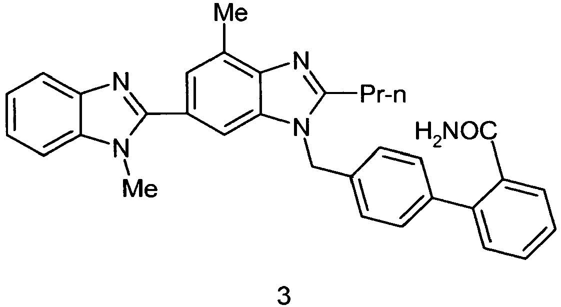 which is hereinafter referred to as carboxamidotelmisartan. The hydrolysis is effected with a base in an organic solvent.

Regardless of the above-discussed preparation processes, there still exists a need for an efficient synthesis of telmisartan and its pharmaceutically acceptable salts. Thus, it is an object of the present invention to provide a process for preparing telmisartan which process is suitable to prepare telmisartan, in particular on an industrial scale, with high quality and high yield using mild reaction conditions in an environmentally friendly as well as cost and time effective way avoiding expensive specialty equipment and rigorous production conditions. This object is surprisingly achieved by the process for preparing telmisartan described herein below.

Telmisartan is commercially available in particular in its free acid form, which is poorly soluble in neutral or acidic media. Thus, telmisartan is typically formulated together with a basic agent or in the form of a basic salt for improved solubility.

Diuretics such as amiloride, chlorothalidone, furosemide, hydrochlorothiazide, indapamide or piretanide are therapeutic agents which can also be used in the treatment of hypertension. It is also known to use combinations of angiotensin II receptor antagonists and diuretics in the treatment of hypertension in order to achieve a synergistic effect. It is generally desirable for such combination formulations that the diuretic is dissolved more rapidly than the angiotensin II receptor antagonist.

Hydrochlorothiazide (HCTZ), the chemical name of which is 6-chloro-1,1-dioxo-3,4-dihydro-2H-1,2,4-benzothiadiazine-7-sulfonamide, is a diuretic which is stable in neutral and relatively weak acidic environments, but poorly stable in alkaline environments. In particular, HCTZ is incompatible with basic agents that are commonly used together with the free acid form of telmisartan. This incompatibility is problematic where combination formulations of telmisartan and a base-labile diuretic such as HCTZ are concerned.

Different approaches have been pursued in order to address the incompatibility problem. A classical approach is to coat diuretic particles with water soluble polymers like hydroxypropylcellulose, hydroxypropylmethylcellulose or polyvinylpyrrolidone. However, this measure has been found not to sufficiently protect the diuretic from degradation when formulated together with telmisartan as a compressed tablet. Moreover, the polymeric coating typically results in a reduced dissolution rate of the diuretic.

WO 2003/059327 A1 discloses a bilayer tablet comprising a first tablet layer containing telmisartan in a dissolving tablet matrix and a second tablet layer containing a diuretic in a disintegrating tablet matrix. WO 2004/028505 A1, WO 2004/096215 A1, WO 2006/063737 A1 and WO 2007/060170 A2 describe further embodiments of bilayer tablets. The telmisartan containing layer is based on a composition that is generally prepared using spray-drying or fluid bed granulation. The diuretic containing layer is based on a composition that is particularly prepared by wet granulation of a diuretic with the constituents of a disintegrating tablet matrix. Among these constituents are a number of specialty excipients such as dry binders, wet granulation binders, and disintegrants.

WO 2007/144175 A2 discloses a pharmaceutical composition comprising first units in the form of granules, pellets or tablet cores comprising telmisartan and second units in the form of granules, pellets, or tablet cores comprising HCTZ. The first and second units can be compressed into tablets together with a suitable carrier such that the units are substantially evenly distributed throughout the tablets. The first and second units are optionally coated. In particular, a separating coating of the first units is contemplated. Furthermore, polyvinylpyrrolidone should be incorporated into the HCTZ containing part of the composition in order to achieve better stability.

In the pharmaceutical compositions of the prior art, complex formulations of the angiotensin II receptor antagonist and the diuretic are necessary to address the incompatibility of the active agents and stabilize the compositions. These complex formulations require the use of substantial amounts of expensive specialty excipients. Moreover, preparation of these formulations involves complex and costly processing steps such as wet granulation.

It is therefore desirable to provide a multilayer pharmaceutical tablet comprising telmisartan and a diuretic in a fixed dosage form avoiding the above-discussed drawbacks and which not only exhibits high chemical and physical stability as well as an appropriate dissolution profile and bioavailability, but which also can be prepared from inexpensive excipients and can be manufactured using a simple and economical process.

This object is surprisingly achieved by the multilayer pharmaceutical tablet described herein below. Also disclosed is a process for the preparation of a multilayer pharmaceutical tablet comprising telmisartan and a diuretic.

### Summary of the invention

In one aspect, the present invention provides a process for the preparation of telmisartan and its pharmaceutically acceptable salts comprising the following steps:
(a) providing a mixture comprising a derivative of telmisartan, an acid and a solvent, wherein the derivative of telmisartan is cyanotelmisartan or carboxamidotelmisartan and the acid is H₂SO₄,
(b) heating the obtained mixture,
(c) optionally converting the telmisartan to its pharmaceutically acceptable salt, and
(d) isolating telmisartan or its pharmaceutically acceptable salt.

### Detailed description of the invention

According to the first aspect, the present invention provides a process for the preparation of telmisartan or a pharmaceutically acceptable salt thereof comprising
(a) providing a mixture comprising a derivative of telmisartan, an acid and a solvent, wherein the derivative of telmisartan is cyanotelmisartan or carboxamidotelmisartan and the acid is H₂SO₄,
(b) heating the obtained mixture,
(c) optionally converting the telmisartan to a pharmaceutically acceptable salt thereof, and
(d) isolating telmisartan or its pharmaceutically acceptable salt.

The derivative of telmisartan used in step (a) is a telmisartan intermediate substituted on position 2 of the biphenyl group of 4'-[(2-n-propyl-4-methyl-6-(1-methylbenzimidazol-2-yl)benzimidazol-1-yl)methyl]biphenyl. The substituent on position 2 is a group Z which can be converted to a carboxyl group. Z is selected from the group consisting of CN and CON(R)_{2,} wherein R is H. Accordingly, the telmisartan derivative used in step (a) is 4'-((1,7'-dimethyl-2'-propyl-1H,3'H-2,5'-bibenzo-[d]imidazol-3'-yl)methyl)biphenyl-2-carbonitrile (cyanotelmisartan) or 4'-((1,7'-dimethyl-2'-propyl-1H,3'H-2,5'-bibenzo[d]imidazol-3'-yl)methyl)biphenyl-2-carboxamide (carboxamidotelmisartan). Preferably, the derivative is cyanotelmisartan. The telmisartan derivative can be prepared by any method known from the prior art, such as for example the methods disclosed in EP 0 502 314 A1, CN 1412183, WO 2004/087676, US 2006/0264491, WO 2007/147889 and WO 2009/004064. If the derivative is cyanotelmisartan, it is preferably prepared by the process according to the third aspect as described below.

In a preferred embodiment, the derivative of telmisartan is used in such an amount that the concentration of the derivative of telmisartan in the mixture of step (a) is more than about 1 mol/L, preferably more than 1.5 mol/L, more preferably more than 1.8 mol/L like about 2 mol/L. Due to the rather high concentration of the starting material the reactor to be used in the production plant may have a rather small volume.

The acid used in step (a) is H₂SO₄. In one embodiment, the acid used in step (a) does not comprise substantially amounts of an organic acid, i.e. the acid comprises less than 10 mol.-%, preferably less than 5 mol.-%, more preferably less than 2 mol.-% and most preferably less than 1 mol.-% of an organic acid, based on the total molar amount of acid used in step (a). The term "organic acid" as used herein refers to any organic compound having at least one carboxyl group -COOH.

The concentration of the acid in the mixture of step (a) is preferably more than about 3 mol/L, more preferably more than 6 mol/L.

Furthermore, it is preferred that the molar ratio between the acid and the derivative of telmisartan ranges from about 2 to about 12, more preferably from 3 to 8, and most preferably from 4 and 6.

The solvent of step (a) can be a polar organic solvent selected from the group consisting of, but not limited to, DMSO, *N,N*-dimethyformamide, *N;N*-dimethylacetamide, alcohols, such as for example butanol, amyl alcohol, isoamyl alcohol or hexylalcohol, water and any mixture thereof. Preferably, the solvent used in step (a) comprises water. In particular, the solvent comprises more than 90 vol.-%, preferably more than 93 vol.-%, more preferably more than 95 vol.-% and even more preferably more than 97 vol.-% of water. Most preferably, the solvent is water.

In step (b), the mixture provided in step (a) is heated. The heating can be performed at temperature between 80 and 150°C, preferably between 100 and 140°C, more preferably between 115 and 130°C. Suitably, the heated mixture is stirred in step (b). Preferably, the mixture is heated for at least 15 hours, such as for 20 to 30 hours in order to complete the hydrolysis reaction. During step (b) the derivative of telmisartan is converted into telmisartan. Thus, at the end of step (b) telmisartan is obtained.

In step (c), the obtained telmisartan is optionally converted into a pharmaceutically acceptable salt thereof. This conversion can be performed by any known method, such as for example the methods disclosed in WO 2007/147889 and WO 2009/004064. Suitable pharmaceutically acceptable salts are selected from the group consisting of, but not limited to, alkaline salts, such as for example sodium or potassium, and amine salts, such as for example lysine, arginine or meglumine, in any crystalline or amorphous form. In another embodiment, the pharmaceutically acceptable salts can be any salts known from the art such as disclosed in WO 03/0307876, CN 1548421, WO 2006/044754, WO 2006/050509, WO 2006/050921, EP 1719766, WO 2006/136916, WO 2007/010559, WO 2007/147889.

Isolating step (d) can be performed by any known method, such as for example the methods disclosed in WO 2007/147889 and WO 2009/004064, or by the process for isolating telmisartan in accordance with the second aspect of the invention as described below.

The process of the present invention allows the preparation of telmisartan and its pharmaceutically acceptable salts in high yields and high purity in a cost efficient manner. It has several advantages over the processes known in the art.

It has surprisingly been found that due to the acidic conditions used in step (a) the heating temperature in step (b) can be lower compared with prior art processes. This results in milder reaction conditions with no special equipment being required. This appears particularly important for the production on bigger scale. Thus, no special high temperature plant for performing the hydrolysis reaction is needed.

Moreover, due to the use of an acid in step (a) the reaction solvent can be water without substantial amounts of high boiling organic solvents like ethylene glycol resulting in an environmentally more friendly process.

In addition, it has surprisingly been found that the use of a rather high concentration of telmisartan intermediate and acid in step (a) allows the preparation of telmisartan in high yield and purity. Such a rather high concentration of the starting materials results in that a smaller reactor volume can be used in the production plant.

Also, it has unexpectedly been found that telmisartan can be prepared by the process of the present invention in high yield and purity, even if the acid used in step (a) does not comprise substantially amounts of an organic acid. In general, organic acids are more expensive than mineral acids and, therefore, the avoidance of organic acids is particularly cost efficient.

In a second aspect, a process for isolating telmisartan from a telmisartan containing mixture is disclosed. This process comprises the steps of:
(a) providing a telmisartan containing mixture,
(b) adjusting pH,
(c) extracting telmisartan with a first organic solvent,
(d) removing the first organic solvent,
(e) crystallizing telmisartan from a second organic solvent, and
(f) optionally converting the telmisartan to its pharmaceutically acceptable salts.

In step (a) a mixture containing telmisartan or a salt thereof is provided. In particular, the mixture can be provided by a process according to the first aspect of the invention. Accordingly, the present invention also relates to a process for the preparation of telmisartan or its pharmaceutically acceptable salts comprising the following steps:
(a') providing a mixture comprising a derivative of telmisartan, an acid and a solvent, wherein the derivative of telmisartan is cyanotelmisartan or carboxamidotelmisartan and the acid is H₂SO₄,
(a'') heating the obtained mixture,
(a''') cooling the obtained mixture,
(b) adjusting pH,
(c) extracting telmisartan with a first organic solvent,
(d) removing the first organic solvent,
(e) crystallizing telmisartan from a second organic solvent, and
(f) optionally converting the telmisartan to its pharmaceutically acceptable salts.

Preferred embodiments of steps (a') and (a'') with regard to telmisartan derivative, acid and solvent and their amounts as well as to reaction conditions are described above in connection with the first aspect of the invention.

It is preferred that in step (a''') the reaction mixture is cooled to a temperature between 0 and 100°C, preferably between room temperature and 80°C.

The mixture of step (a) can also be provided by preparing telmisartan or a salt thereof according to any process known from the state of the art. Thus, telmisartan can also be prepared by a process for the preparation of telmisartan or its pharmaceutically acceptable salts comprising the following steps:
(a') providing a mixture comprising a salt of telmisartan prepared by any process known from the state of the art,
(b) adjusting pH,
(c) extracting telmisartan with a first organic solvent,
(d) removing the first organic solvent,
(e) crystallizing telmisartan from a second organic solvent, and
(f) optionally converting the telmisartan to its pharmaceutically acceptable salts.

In step (b) of the process according to the second aspect, the pH of the mixture obtained in step (a) is adjusted. Preferably, the pH is adjusted to 4 to 7 and more preferably to 4.5 to 6. It is particularly preferred to adjust the pH to 5 to 5.5. Accordingly, the adjusted pH of the mixture provided in step (a') comprising the derivative of telmisartan, the acid and the solvent is preferably between 4 and 7, more preferably between 4.5 and 6. It is preferred that in step (b) the pH is adjusted by using an aqueous solution of mineral bases such as for example NaOH, KOH, LiOH, Ca(OH)₂ and the like, mineral acids such as for example HCl and H₂SO₄, ammonia or organic bases such as for example amines. Preferably, NaOH or KOH is used for pH adjustment.

Extraction of telmisartan in step (c) can be performed with a first organic solvent selected from the group consisting of, but not limited to, an ester such as for example ethyl acetate, isopropyl acetate or butyl acetate, an ether such as for example diethyl ether, diisopropyl ether or tert-buty methyl ether, toluene, a halogenated organic solvent such as for example dichloromethane and any mixtures thereof. Preferably, dichloromethane is used for extracting telmisartan.

In step (d), the first organic solvent can be removed by evaporation or any other method known from the art.

The second organic solvent used for crystallizing telmisartan in step (e) can be selected from the group consisting of, but not limited to, an alcohol such as for example methanol, ethanol or isopropanol, an ester such as for example ethyl acetate, isopropyl acetate or butyl acetate, a ketone such as for example acetone, 2-butanone, 3-pentanone or methyl isobutyl ketone, an ether such as for example diethyl ether, diisopropyl ether or tert-buty methyl ether, a nitrile such as for example acetonitrile and any mixtures thereof. Preferably, acetone is used as second organic solvent.

In optional step (f), the telmisartan obtained in step (e) can be converted into a pharmaceutically acceptable salt thereof. This optional conversion can be performed by any known method, such as for example the methods disclosed in WO 2007/147889 and WO 2009/004064. The pharmaceutically acceptable salts can be selected from the group consisting of, but not limited to, alkaline salts, such as for example sodium or potassium, and amine salts, such as for example lysine, arginine or meglumine, in any crystalline or amorphous form. In another embodiment, the pharmaceutically acceptable salts can be any salts known from the art such as disclosed in WO 03/0307876, CN 1548421, WO 2006/044754, WO 2006/050509, WO 2006/050921, EP 1719766, WO 2006/136916, WO 2007/010559, WO 2007/147889.

The purity of the telmisartan or its pharmaceutically acceptable salts obtained by the process according to the second aspect is more than 99.0%, preferably more than 99.5%, according to Area% HPLC method.

Thus, it has surprisingly been found that the process of the second aspect provides the desired product in high yields and a high purity.

In particular, if in step (a) of the process according to the second aspect the mixture comprising telmisartan or a salt thereof is provided by the process according to the first aspect of the invention, the rather low excess of acid used in (a') results in that no high amounts of base are needed for adjusting the pH in step (b). In addition, the side product of the pH adjusting step can be removed by the water phase during the extraction of the product with an organic solvent in step (c).

Moreover, in view of extracting step (c) there is no need for evaporation of the reaction solvent. This has proven to be particularly beneficial since the first organic solvent used for extraction has usually a lower boiling point than the reaction solvent.

According to a third aspect, cyanotelmisartan (4'-((1,7'-dimethyl-2'-propyl-1H,3'H-[2,5'-bibenzo[d]imidazol]-3'-yl)-methyl)-[1,1'-biphenyl]-2-carbonitrile) can be prepared by a process comprising
(a) reacting 1,7'-dimethyl-2'-propyl-1H,3'H-2,5'-bibenzo[d]-imidazole or a salt thereof with 4'-(halomethyl)-(1,1'-biphenyl]-2-carbonitrile in the presence of a base and a solvent to obtain cyanotelmisartan.

The starting materials, i.e. 1,7'-dimethyl-2'-propyl-1H,3'H-2,5'-bibenzo[d]imidazole or a salt thereof, 4'-(halomethyl)-(1,1'-biphenyl]-2-carbonitrile, base and solvent, are preferably mixed and the resulting mixture is stirred. Suitably, the mixture is stirred for 0.5 to 24 hours, preferably 0.5 to 2 hours such as about 1 hour. The reaction temperature can range from about 10 to 80°C, preferably from about 15 to 30°C such as room temperature.

Suitable bases include, but are not limited to, alkali metal bases such as alkali metal bases selected from the group consisting of LiOH, NaOH, K₂CO₃, Na₂CO₃, Li₂CO₃, Cs₂CO₃ and KOH. Preferably, the base is KOH.

Suitable solvents include, but are not limited to, polar organic solvents selected from the group consisting of *N,N-*dimethylformamide, *N*,*N*-dimethylacetamide, acetonitrile, dimethylsulfoxide and mixtures thereof. According to a preferred embodiment, the solvent comprises acetonitrile and more preferably the solvent is acetonitrile.

It is preferred that 4'-(bromomethyl)-[1,1'-biphenyl]-2-carbonitrile is used as 4'-(halomethyl)-[1,1'-biphenyl]-2-carbonitrile. Moreover, it is preferred that the molar ratio of 4'-(halomethyl)-[1,1'-biphenyl]-2-carbonitrile to 1,7'-dimethyl-2'-propyl-1H,3'H-2,5'-bibenzo[d]imidazole or its salt is 1.2 to 1.0. In addition, the molar ratio of base to 1,7'-dimethyl-2'-propyl-1H,3'H-2,5'-bibenzo[d]imidazole or its salt is preferably 1.8 to 1.0 such as 1.5 to 1.3.

After completion of the reaction, the reaction mixture can be cooled to a temperature of about -10 to 10°C, preferably about -5 to 5°C like about 0°C. The formed precipitate can be separated from the reaction mixture by e.g. filtration. Furthermore, the isolated cyanotelmisartan can be washed with water and/or an organic solvent such as alcohols, nitriles or ketones and dried. Preferably, the isolated cyanotelmisartan is washed with water and dried.

The obtained product can be further purified. For example, it can be recrystallized from an organic solvent such as acetonitrile, *N,N-* dimethylformamide or *N*,*N*- dimethylacetamide. Also, the product can be suspended in an organic solvent such as for example acetonitrile, ethyl acetate or isopropyl acetate.

It has surprisingly been found that cyanotelmisartan can be obtained in a high yield and purity by the process according to the third aspect.

The cyanotelmisartan prepared by the above process can be further reacted to telmisartan by any method known in the art, such as WO 2004/087676, CN 1412183 or WO 2009/004064, or by the process according to the first aspect of the present invention or the second aspect described above. Accordingly, in a preferred embodiment the invention provides a process for preparing telmisartan or a pharmaceutically acceptable salt thereof comprising
(a) reacting 1,7'-dimethyl-2'-propyl-1H,3'H-2,5'-bibenzo[d]-imidazole or a salt thereof with 4'-(halomethyl)-[1,1'-biphenyl]-2-carbonitrile in the presence of a base and a solvent to obtain cyanotelmisartan,
(b) providing a mixture comprising the obtained cyanotelmisartan, an acid and a solvent, wherein the acid is H₂SO₄,
(c) heating the obtained mixture,
(d) optionally converting the telmisartan to a pharmaceutically acceptable salt thereof, and
(e) isolating telmisartan or its pharmaceutically acceptable salt.

Preferred embodiments of steps (a) and (e) are described above in connection with the first aspect of the invention and the third aspect described above, respectively.

Furthermore, in another preferred embodiment a process for preparing telmisartan or a pharmaceutically acceptable salt thereof is disclosed comprising
(a') reacting 1,7'-dimethyl-2'-propyl-1H,3'H-2,5'-bibenzo[d]-imidazole or a salt thereof with 4'-(halomethyl)-[1,1'-biphenyl]-2-carbonitrile in the presence of a base and a solvent to obtain cyanotelmisartan,
(a'') providing a mixture comprising the obtained cyanotelmisartan, an acid and a solvent,
(a"') heating the obtained mixture,
(a'''') cooling the obtained mixture,
(b) adjusting pH,
(c) extracting telmisartan with a first organic solvent,
(d) removing the first organic solvent,
(e) crystallizing telmisartan from a second organic solvent, and
(f) optionally converting the telmisartan to its pharmaceutically acceptable salts.

Preferred embodiments of steps (a') to (f) are described above in connection with the first aspect of the invention as well as the second and third aspect described above.

In a fourth aspect, a multilayer pharmaceutical tablet is disclosed comprising
(a) at least one first tablet layer comprising
   1 to 50 wt.-% telmisartan or a pharmaceutically acceptable salt thereof
   by weight of the first tablet layer; and
(b) at least one second tablet layer comprising
   1 to 50 wt.-% of a diuretic and
   50 to 99 wt.-% of at least one filler
   by weight of the second tablet layer, wherein the combined weight of the diuretic and the at least one filler is at least 87 wt.-% by weight of the second tablet layer.

As used herein, the term "multilayer pharmaceutical tablet" refers to a pharmaceutical tablet which is made up of at least two distinct layers, such as at least two layers, at least three layers, at least four layers, at least five layers, etc., with the individual layers being arranged one on top of another. The multilayer tablet generally has a sandwich-like appearance because the edges of each layer are exposed. Typically, adjacent layers of the tablet will be of different composition. It has to be understood that the terms "first tablet layer" and "second tablet layer" as used herein refer to tablet layers having a particular composition. However, these terms do not necessarily reflect the order in which the layers are arranged in the tablet.

It has surprisingly been found that the multilayer pharmaceutical tablet exhibits a dissolution profile that is comparable to the one typically found for tablets containing telmisartan in a dissolving tablet matrix and a diuretic in a disintegrating tablet matrix.

The first tablet layer comprises telmisartan or a pharmaceutically acceptable salt thereof. Telmisartan is typically employed in its free acid form although pharmaceutically acceptable salts can also be used. The first tablet layer preferably comprises 3 to 50 wt.-%, particularly 5 to 35 wt.-%, more preferably 10 to 20 wt.-% of telmisartan or a pharmaceutically acceptable salt thereof.

According to one embodiment, the first tablet layer comprises telmisartan and a basic agent. The term "basic agent" as used herein refers to a substance which is characterized in that a 3 wt.-% aqueous solution thereof has a pH value of at least 8.0. Suitable basic agents include ammonia, NaOH, KOH, Ca(OH)₂, Na₂CO₃, K₂CO₃, NaHCO₃, KHCO₃, Na₃PO₄, K₃PO₄, Na₂HPO₄, K₂HPO₄, choline, tert-butylamine, ethanolamine, meglumine, piperazine, diethylamine, L-arginine and mixtures thereof. Alkali metal hydroxides such as NaOH and KOH, amino sugars such as meglumine and mixtures thereof are preferred basic agents. It is particularly preferred that the basic agent comprises a mixture of an alkali metal hydroxide such as NaOH or KOH and an amino sugar such as meglumine in a weight ratio of 1:1 to 1:10, more particularly 1:2 to 1:5, more preferably 1:3 to 1:4, most preferably about 1:3.5. The first tablet layer preferably comprises 0.25 to 20 wt.-%, particularly 1 to 15 wt.-%, more preferably 2 to 10 wt.-% of basic agent.

The telmisartan can be amorphous or crystalline. As used herein, the term "amorphous" includes amorphous and partly crystallized forms. Amorphous telmisartan can be obtained by methods generally known in the art such as freeze drying of aqueous solutions, coating of carrier particles by a solution of telmisartan in a fluidized bed, and solvent deposition on sugar pellets or other carriers.

According to another embodiment, the first tablet layer comprises a pharmaceutically acceptable salt of telmisartan. Suitable pharmaceutically acceptable salts include alkaline salts such as the sodium salt of telmisartan. The pharmaceutically acceptable salt of telmisartan can be amorphous or crystalline.

In addition to telmisartan or a pharmaceutically acceptable salt thereof, the first tablet layer can comprise pharmaceutically acceptable excipients. The term "pharmaceutical acceptable excipient" as used herein refers to additives useful for converting pharmacologically active compounds into pharmaceutical dosage forms which are suitable for administration to patients. Excipients suitable for use in the first tablet layer include fillers, binders, surfactants, crystallization retarders, lubricants, glidants and coloring agents. Other pharmaceutically acceptable excipients can also be included.

The first tablet layer typically comprises at least one filler. Water-soluble fillers are generally preferred. Suitable fillers for use in the first tablet layer include monosaccharides, oligosaccharides and sugar alcohols such as glucose, fructose, saccharose, lactose (anhydrous and monohydrate), raffinose, trehalose, dextrates, mannitol, erythritol, sorbitol, maltitol, xylitol and lactitol, compressible sugar, calcium hydrogen phosphate, calcium carbonate, calcium lactate and mixtures thereof. Preferred fillers are monosaccharides and oligosaccharides such as glucose, fructose, saccharose and lactose, sugar alcohols such as mannitol, erythritol, sorbitol, maltitol, xylitol and lactitol and mixtures thereof. Lactose, sorbitol and mixtures thereof are particularly preferred. It is particularly preferred that the filler comprises a monosaccharide or oligosaccharide such as lactose and a sugar alcohol such as sorbitol in a weight ratio of 1:1 to 1:10, particularly 1:2 to 1:5, most preferably about 1:2.5. The first tablet layer preferably comprises 30 to 95 wt.-%, particularly 60 to 90 wt.-%, more preferably 70 to 80 wt.-% of filler.

Suitable binders for use in the first tablet layer include povidone (polyvinylpyrrolidone), copovidone (vinylpyrrolidonevinylacetate copolymer), cellulose powder, crystalline cellulose, microcrystalline cellulose, siliconized microcrystalline cellulose, cellulose derivatives such as hydroxymethylcellulose, hydroxyethylcellulose, hydroxypropylcellulose and hydroxypropylmethylcellulose, starch, pregelatinized starch, polymethacrylates and mixtures thereof. Povidone is particularly preferred. The first tablet layer preferably comprises 1 to 30 wt.-%, particularly 2 to 10 wt.-%, more preferably 3 to 7 wt.-% of binder.

Suitable surfactants for use in the first tablet layer include anionic, cationic, ampholytic and non-ionic surfactants such as sodium lauryl sulfate, cetrimide, N-dodecyl-N,N-dimethylbetaine, polysorbates (such as Tweens®), poloxamers and mixtures thereof. Non-ionic surfactants such as polysorbates and poloxamers are preferred. The first tablet layer preferably comprises 1 to 30 wt.-%, particularly 2 to 10 wt.-%, more preferably 3 to 7 wt.-% of surfactant.

Suitable crystallization retarders for use in the first tablet layer include povidone, copovidone, crospovidone, carboxymethylcellulose sodium, hydroxypropylcellulose and hydroxypropylmethylcellulose. The first tablet layer preferably comprises 0.1 to 10 wt.-%, particularly 0.25 to 5 wt.-%, more preferably 0.5 to 2 wt.-% of crystallization retarder.

Suitable lubricants for use in the first tablet layer include stearic acid and stearic acid salts such as magnesium stearate, magnesium palmitate and magnesium oleate, hydrogenated vegetable oil, hydrogenated castor oil, talc, sodium stearyl fumarate, macrogols and mixtures thereof. Stearic acid, magnesium stearate and hydrogenated vegetable oil are particularly preferred. The first tablet layer preferably comprises 0.1 to 10 wt.-%, particularly 0.25 to 5 wt.-%, more preferably 0.5 to 2 wt.-% of lubricant.

Suitable glidants for use in the first tablet layer include colloidal silicon dioxide and magnesium trisilicate. The first tablet layer preferably comprises 0.1 to 10 wt.-%, particularly 0.25 to 5 wt.-%, more preferably 0.5 to 2 wt.-% of glidant.

Suitable coloring agents for use in the first tablet layer include dyes and pigments such as iron oxide and titanium oxide. The first tablet layer preferably comprises 0.001 to 1 wt.-%, particularly 0.01 to 0.5 wt.-%, more preferably 0.05 to 0.2 wt.-% of coloring agent.

According to a particular embodiment, the first tablet layer comprises
(aa) 3 to 50 wt.-%, particularly 5 to 35 wt.-%, more preferably 10 to 20 wt.-% of telmisartan or a pharmaceutically acceptable salt thereof;
(bb) 0.25 to 20 wt.-%, particularly 1 to 15 wt.-%, more preferably 2 to 10 wt.-% of basic agent;
(cc) 30 to 95 wt.-%, particularly 60 to 90 wt.-%, more preferably 70 to 80 wt.-% of filler;
(dd) 1 to 30 wt.-%, particularly 2 to 10 wt.-%, more preferably 3 to 7 wt.-% of binder;
(ee) 0 to 30 wt.-%, particularly 2 to 10 wt.-%, more preferably 3 to 7 wt.-% of surfactant;
(ff) 0 to 10 wt.-%, particularly 0.25 to 5 wt.-%, more preferably 0.5 to 2 wt.-% of lubricant; and
(gg) 0 to 1 wt.-%, particularly 0.01 to 0.5 wt.-%, more preferably 0.05 to 0.2 wt.-% of coloring agent.

The second tablet layer comprises a diuretic and at least one filler. Preferred diuretics include amiloride, chlorothalidone, furosemide, hydrochlorothiazide, indapamide or piretanide. Hydrochlorothiazide (HCTZ) is particularly preferred.

The diuretic has preferably an average particle size of less than 80 µm, particularly less than 50 µm, more preferably less than 30 µm. The term "average particle size" as used herein refers to the volume mean diameter of particles. The volume mean diameter can be determined by laser light scattering for instance using a Malvern Mastersizer Apparatus MS 2000 equipped with a Hydro S dispersion unit.

The second tablet layer comprises at least one filler, wherein the combined weight of the diuretic and the at least one filler is at least 87 wt.-% by weight of the second tablet layer. It is particularly preferred that the combined weight of the diuretic and the filler is at least 90 wt.-%, more preferably at least 93 wt.-%, further more preferably at least 95 wt.-%, most preferably at least 99 wt.-% by weight of the second tablet layer.

Water-soluble fillers are generally preferred. Suitable fillers for use in the second tablet layer include monosaccharides, oligosaccharides and sugar alcohols such as glucose, fructose, saccharose, lactose (anhydrous and monohydrate), raffinose, trehalose, dextrates, mannitol, erythritol, sorbitol, maltitol, xylitol and lactitol, compressible sugar, calcium hydrogen phosphate, calcium carbonate, sodium carbonate in anhydrous or hydrated form, sodium hydrogen carbonate, calcium lactate and mixtures thereof. Preferred fillers are monosaccharides and oligosaccharides such as glucose, fructose, saccharose and lactose, sugar alcohols such as mannitol, erythritol, sorbitol, maltitol, xylitol and lactitol and mixtures thereof. Mannitol is particularly preferred. The second tablet layer preferably comprises 50 to 99 wt.-%, particularly 80 to 98 wt.-%, more preferably 90 to 96 wt.-% of filler.

The second tablet layer can generally comprise additional pharmaceutically acceptable excipients. Additional excipients suitable for use in the second tablet layer include binders, surfactants, lubricants, glidants and coloring agents.

Suitable binders for use in the second tablet layer include povidone (polyvinylpyrrolidone), copovidone (vinylpyrrolidonevinylacetate copolymer), polyvinyl alcohol, graft copolymer of polyethyleneglycol and polyvinyl alcohol obtainable as Kollicoat IR, polyethylene glycol having a molecular weight in the range of 200 to 10,000, preferably in the range 1,000 to 8,000, cellulose powder, crystalline cellulose, microcrystalline cellulose, siliconized microcrystalline cellulose, cellulose derivatives such as hydroxymethylcellulose, hydroxyethylcellulose, hydroxypropylcellulose and hydroxypropylmethylcellulose, starch, pregelatinized starch, polymethacrylates and mixtures thereof. In particular, the second tablet layer can comprise 1 to 20 wt.-%, particularly 2 to 10 wt.-%, more preferably 3 to 7 wt.-% of binder. Preferably, water soluble binders such as povidone (polyvinylpyrrolidone), copovidone (vinylpyrrolidone-vinylacetate copolymer), polyvinyl alcohol, graft copolymer of polyethyleneglycol and polyvinyl alcohol obtainable as Kollicoat IR, polyethyleneglycol with molecular weight in the range of 200 to 10,000, preferably in the range 1,000 to 8,000, hydroxypropylmethylcellulose having a viscosity (2 wt/vol% solution in water at 25°C) of less than 50 mPas, preferably of less than 15 mPas can be used.

Suitable surfactants for use in the second tablet layer include anionic, cationic, ampholytic and non-ionic surfactants such as sodium lauryl sulfate, cetrimide, N-dodecyl-N,N-dimethylbetaine, polysorbates (such as Tweens®), poloxamers and mixtures thereof. Non-ionic surfactants such as polysorbates and poloxamers are preferred. In particular, the second tablet layer can comprise 1 to 30 wt.-%, particularly 2 to 10 wt.-%, more preferably 3 to 7 wt.-% of surfactant.

Suitable lubricants for use in the second tablet layer include stearic acid or stearic acid salts, such as magnesium stearate, magnesium palmitate and magnesium oleate, hydrogenated vegetable oil, hydrogenated castor oil, talc, sodium stearyl fumarate, macrogols and mixtures thereof. Stearic acid, magnesium stearate and hydrogenated vegetable oil are particularly preferred. In particular, the second tablet layer can comprise 0.1 to 10 wt.-%, particularly 0.25 to 5 wt.-%, more preferably 0.5 to 2 wt.-% of lubricant.

Suitable glidants for use in the second tablet layer include colloidal silicon dioxide and magnesium trisilicate. In particular, the second tablet layer can comprise 0.1 to 10 wt.-%, particularly 0.25 to 5 wt.-%, more preferably 0.5 to 2 wt.-% of glidant.

Suitable coloring agents for use in the second tablet layer include dyes and pigments such as iron oxide and titanium oxide. In particular, the second tablet layer can comprise 0.001 to 1 wt.-%, particularly 0.01 to 0.5 wt.-%, more preferably 0.05 to 0.2 wt.-% of coloring agent.

According to a preferred embodiment, the second tablet layer comprises

| | |
|---|---|
| (aa) | 1 to 50 wt.-%, particularly 2 to 20 wt.-%, more prefera-bly 4 to 8 wt.-% of a diuretic, preferably HCTZ; |
| (bb) | 50 to 99 wt.-%, particularly 80 to 98 wt.-%, more preferably 90 to 96 wt.-% of filler; |
| (cc) | 0 to 30 wt.-%, particularly 2 to 10 wt.-%, more preferably 3 to 7 wt.-% of surfactant; |
| (dd) | 0 to 10 wt.-%, particularly 0.25 to 5 wt.-%, more preferably 0.5 to 2 wt.-% of lubricant; |
| (ee) | 0 to 10 wt.-%, particularly 0.25 to 5 wt.-%, more preferably 0.5 to 2 wt.-% of glidant; and |
| (ff) | 0 to 1 wt.-%, particularly 0.01 to 0.5 wt.-%, more preferably 0.05 to 0.2 wt.-% of coloring agent. |

Other pharmaceutically acceptable excipients can also be included in the second tablet layer. However, it is generally preferred that the second tablet layer does not comprise substantial amounts of disintegrant. It is further preferred that the second tablet layer comprises less than 2 %, particularly less than 1 %, more preferably less than 0.5 %, most preferably less than 0.1 % by weight of the second tablet layer of a disintegrant. According to a particularly preferred embodiment, the second tablet layer is substantially free of disintegrant.

The term "disintegrant" as used herein refers to any material that has wicking and/or swelling properties when it comes in contact with water. In particular, the term "disintegrant" refers to the group of compounds consisting of crospovidone, pregelatinized starch, sodium starch glycolate, hydroxypropyl starch, carboxymethylcellulose sodium and calcium, cross-linked carboxymethylcellulose sodium, polacrilin potassium, low-substituted hydroxypropylcellulose, sodium and calcium alginate, docusate sodium, methylcellulose, agar, guar gum, chitosan and alginic acid. More particularly, the term "disintegrant" refers to the group of compounds consisting of povidone, crospovidone, starch, pregelatinized starch, sodium starch glycolate, hydroxypropyl starch, microcrystalline cellulose, carboxymethylcellulose sodium and calcium, cross-linked carboxymethylcellulose sodium, polacrilin potassium, low-substituted hydroxypropylcellulose, sodium and calcium alginate, docusate sodium, methylcellulose, agar, guar gum, chitosan and alginic acid.

According to a particular embodiment, the second tablet layer consists of a diuretic and at least one filler.

Where the multilayer pharmaceutical tablet comprises more than two layers, various embodiments are possible with respect to the composition and order of arrangement of layers. For instance, where the multilayer pharmaceutical tablet is a three-layer tablet, the tablet comprises an additional layer in addition to a first tablet layer and a second tablet layer as defined above. The additional layer can be a further first tablet layer or a further second tablet layer as defined above. For instance, a three-layer tablet can have one first tablet layer arranged between two second tablet layers or one second tablet layer arranged between two first tablet layers. Alternatively, the additional layer can be a layer differing in composition from both the first tablet layer as well as the second tablet layer. For instance, the additional layer can be a telmisartan containing layer of different composition than the first tablet layer or a diuretic containing layer of different composition that the second tablet layer. It can also be a layer containing neither telmisartan nor diuretic. The same applies mutatis mutandis to a multilayer tablet having more than three layers.

According to one embodiment, the multilayer pharmaceutical tablet is a bilayer tablet having a first tablet layer comprising telmisartan and a second tablet layer comprising a diuretic as defined above. According to another embodiment, the multilayer pharmaceutical tablet is a three-layer tablet having a first tablet layer comprising telmisartan arranged between two second tablet layers comprising a diuretic.

Also disclosed is a multilayer pharmaceutical tablet comprising
(a) at least one first tablet layer comprising
   1 to 50 wt.-% telmisartan or a pharmaceutically acceptable salt thereof by weight of the first tablet layer; and
(b) at least one second tablet layer comprising
   1 to 50 wt.-% of a diuretic; and
   less than 2 wt.-% of disintegrant
   by weight of the second tablet layer.

The term "disintegrant" is to be understood as defined above.

It is particularly preferred that the second tablet layer comprises less than 2 wt.-%, in particular less than 1 wt.-%, more preferably less than 0.5 wt.-%, most preferably less than 0.1 wt.-% by weight of the second tablet layer of one or more disintegrants selected from the group consisting of crospovidone, pregelatinized starch, sodium starch glycolate, hydroxypropyl starch, carboxymethylcellulose sodium and calcium, cross-linked carboxymethylcellulose sodium, polacrilin potassium, low-substituted hydroxypropylcellulose, sodium and calcium alginate, docusate sodium, methylcellulose, agar, guar gum, chitosan and alginic acid. It is even more particularly preferred that the second tablet layer comprises less than 2 wt.-%, in particular less than 1 wt.-%, more preferably less than 0.5 wt.-%, most preferably less than 0.1 wt.-% by weight of the second tablet layer of one or more disintegrants selected from the group consisting of povidone, crospovidone, starch, pregelatinized starch, sodium starch glycolate, hydroxypropyl starch, microcrystalline cellulose, carboxymethylcellulose sodium and calcium, cross-linked carboxymethylcellulose sodium, polacrilin potassium, low-substituted hydroxypropylcellulose, sodium and calcium alginate, docusate sodium, methylcellulose, agar, guar gum, chitosan and alginic acid.

It is further preferred that the second tablet layer comprises less than 1 wt.-%, more preferably less than 0.5 wt.-%, most preferably less than 0.1 wt.-% of disintegrant by weight of the second tablet layer. According to a particularly preferred embodiment, the second tablet layer is substantially or entirely free of disintegrant.

It has been found that the multilayer pharmaceutical tablet of the invention comprising only low amounts of or being substantially free of disintegrant can be formulated very effectively. Surprisingly, the multilayer pharmaceutical tablet thus formulated exhibits a dissolution profile that is comparable to the one typically found for tablets containing telmisartan in a dissolving tablet matrix and a diuretic in a disintegrating tablet matrix.

Further preferred embodiments of the multilayer pharmaceutical tablet with regard to specific components and their amounts are as described above for the first aspect of the invention.

The multilayer pharmaceutical tablet can be prepared in principle by any method used for manufacturing tablet formulations. Preparation of the tablet generally comprises preparing a first tablet layer composition comprising telmisartan and a second tablet layer composition comprising a diuretic and compressing the tablet layer compositions to produce a multilayer tablet.

The first tablet layer composition comprising telmisartan can be prepared by various methods. Suitable methods include spray-drying and fluid-bed granulation.

One method for preparing the first tablet layer composition comprises preparing a spray-solution by dissolving telmisartan together with at least one basic agent in an appropriate solvent (e.g. water or organic solvent). Optionally, additional excipients, such as a crystallization retarder and/or a surfactant can be included in the spray-solution. The spray solution is subsequently spray-dried to give a spray-dried granulate. The spray-dried granulate is mixed with further excipients to give a tablet layer composition ready for tableting.

Another method for preparing the first tablet layer composition comprises preparing a granulation liquid by dissolving telmisartan together with at least one basic agent in an appropriate solvent (e.g. water or organic solvent). Optionally, additional excipients, such as a crystallization retarder and/or a surfactant can be included in the spray-solution. At least one excipient selected from fillers, binders and mixtures thereof is placed into a fluid-bed granulating machine and sprayed with the granulation liquid. When granulation is completed, the granulate is dried and optionally mixed with further excipients to give a tablet layer composition ready for tableting.

The second tablet layer composition comprising a diuretic can also be prepared by various methods such as direct compression, compression of a granulate obtained by state of the art processes like wet, dry or thermoplastic granulation or melt extrusion. For instance, the second tablet layer composition can in a first embodiment of the present invention be prepared using a wet granulation technique. A suitable wet granulation technique comprises preparing a granulation liquid by dissolving at least a part of a filler and/or binder in an appropriate solvent (e.g. water, organic solvent or a mixture thereof) and adding like spraying the granulation liquid onto a premix comprising diuretic and optionally additional excipients. The obtained wet granulate is dried, optionally sieved and optionally mixed with further excipients to give a tablet layer composition ready for tableting.

According to a second embodiment, the second tablet layer composition is prepared using a dry granulation method as described below.

According to a third embodiment, the second tablet layer composition is prepared using a thermoplastic granulation, wherein a mixture of diuretic and least one exipient is granulated with a binder having a melting or softening point below 170°C, preferably below 150°C and most preferably below 130°C. Suitable binders having low melting or softening point can be selected from polymers such as, but not limited to, polyethylene glycol having a molecular weight in the range 1,000 to 10,000, poloxamer, povidone with a K value of less than 50, copovidone, Soluplus^{®} or mixtures thereof. State of the art equipment such as high share mixer, fluid bed granulator or extruder can be used in preparation of thermoplastic granulates. Agglomeration can be achieved by adding melted binder or by in situ melting or softening of a binder having low melting or softening point. In situ melting can be achieved by heating the components to the temperature of at least 5 degrees (°C) above the melting or softening temperature of a binder or by heat release due to mixing the powders or elevated pressure. The obtained granulate can optionally be mixed with further excipients and compressed into multilayer tablet.

According to a fourth embodiment, the second tablet layer composition is prepared using a direct compression method where a powder premix of diuretic and at leas one further exicient selected from filler, binder, lubricant, glidant and mixtures thereof is directly compressed onto the first layer containing telmisartan.

In order to prepare the final multilayer pharmaceutical tablet, the first and second tablet layer compositions can be compressed in a manner generally known in the art, such as using a rotary tablet press in an appropriate multilayer tableting mode. For instance, for the manufacture of a bilayer tablet, the first tablet layer composition comprising telmisartan can be introduced into the tableting machine first, followed by introducing the second tablet layer composition comprising diuretic and compressing the two layers in a bilayer tableting mode. Likewise, for the manufacture of a three layer tablet, part of the second tablet layer composition comprising diuretic can be introduced into the tableting machine first, followed by introducing the first tablet layer composition comprising telmisartan and then the remaining second tablet layer composition comprising diuretic and compressing all three layers in a three layer tableting mode.

A process for the preparation of a multilayer pharmaceutical tablet comprising at least one layer comprising telmisartan and at least one layer comprising a diuretic is also disclosed, the process comprising
(a) providing a first tablet layer composition comprising telmisartan;
(b) providing a second tablet layer composition comprising a diuretic by
   (i) dry granulating the diuretic with at least one filler and optionally other pharmaceutically acceptable excipients to obtain a granulate; and
   (ii) optionally blending the obtained granulate with additional excipients;
(c) optionally providing one or more further tablet layer compositions; and
(d) compressing said tablet layer compositions to result in the multilayer pharmaceutical tablet.

Preferred embodiments of the first tablet layer composition comprising telmisartan with regard to specific components and their amounts as well as to methods for its preparation are as described above.

Preferred embodiments of the second tablet layer composition with regard to specific components and their amounts are as described above.

It has surprisingly been found that a process for the preparation of a multilayer tablet, wherein a dry granulation method is used for preparing the second tablet layer composition comprising a diuretic, provides a number of advantages. Such process is particularly suitable for application on an industrial scale. In particular, the process is simple and highly cost effective. The tablet layer composition ready for tableting which is obtained according to the process exhibits excellent physical characteristics particularly with regard to flowability and provides for very fast dissolution of the diuretic in the final tablet.

In particular, the second tablet layer composition is prepared by mixing the diuretic and at least one filler in a suitable mixer in order to prepare a pre-mixture. The pre-mixture is then compacted on a dry granulation machine and the compact milled into granules of appropriate particle size. It is preferred that the compact is milled into granules having an average particle size of less than 80 µm, particularly less than 50 µm, more preferably less than 30 µm.

In order to prepare the final multilayer pharmaceutical tablet, the first and second tablet layer compositions and optionally further tablet layer compositions are compressed in a multilayer tableting mode as described in more detail above.

Also disclosed is a multilayer pharmaceutical tablet prepared by the process according to the invention.

The invention will be further illustrated by way of the following examples with Examples 4 to 9 not being in accordance with the invention.

### Examples

### Example 1: Preparation and isolation of telmisartan

Into a reaction vessel 20g (40 mmol) cyanotelmisartan and 20 ml (180mmol) H₂SO₄ (1:1) were added. The reaction mixture was heated to around 125°C and stirred at this temperature for 30 h. A sample of the reaction mixture was analyzed by Area% HPLC (cyanotelmisartan below 0.1 %, intermediate amide of telmisartan less than 0,2%, telmisartan over 97%). The reaction mixture was cooled below 80°C and 250 ml of water were added. Then, 200 ml of dichloromethane were added and pH value of mixture was adjusted to 5.3 by addition of 6M NaOH. The mixture was stirred for approximately 5 min and then the phases were separated. The water phase was reextracted with 136 ml of dichloromethane. Collected organic phases were washed with water (2×136ml) and then treated with activated charcoal (5.3 g). Subsequently, the organic phase was evaporated to an oily residue (26g). 264 ml of acetone were added. The mixture was stirred at room temperature for at least 6 hours. The precipitated product was separated and washed with fresh acetone and dried at 65°C under reduced pressure for 3 hours.
Yield: 18g (88%)
Area % HPLC: Telmisartan 99.86%

### Example 2: Preparation and isolation of telmisartan

Into a reaction vessel 20.5g (40 mmol) 4'-((1,7'-dimethyl-2'-propyl-1H,3'H-2,5'-bibenzo[d]imidazol-3'-yl)methyl)biphenyl-2-carboxamide and 20 ml (180mmol) H₂SO₄ (1:1) were added. The re action mixture was heated to about 125°C and stirred at this temperature for 28 h. A sample of the reaction mixture was analyzed by Area% HPLC (starting compound below 0.1%, telmisartan over 97%). The reaction mixture was cooled below 80°C and 250 ml of water were added. Then, 200 ml of dichloromethane were added and pH value of mixture was adjusted to 5.4 by addition of 6M NaOH. The mixture was stirred for approximately 5 min and then the phases were separated. The water phase was reextracted by 136 ml of dichloromethane. Collected organic phases were washed with water (2x136ml) and then treated with activated charcoal (5.3 g). Subsequently, the organic phase was evaporated an oily residue (26g). 264 ml of acetone were added. The mixture was stirred at room temperature for at least 6 hours. The precipitated product was separated and washed with fresh acetone and dried at 65°C under reduced pressure for 3 hours.
Yield: 18.3g (89%)
Area % HPLC: Telmisartan 99.80%

### Example 3: Isolation of telmisartan

Into a reaction vessel 7.5g (15 mmol) of cyanotelmisartan, 30 ml of propylene glycol, 0.8 ml of water and 3g (45 mmol) of 85% KOH were added. The reaction mixture was heated to around 160°C to 170°C and stirred at this temperature for 24 h. The reaction mixture was cooled below 80°C and 75 ml of water were added. Then, pH value of the mixture was adjusted to 4.8 (by addition of 6M HCl) and then 150 ml of dichloromethane were added. The mixture was stirred for approximately 5 min and then the phases were separated. The water phase is reextracted by 50 ml of dichloromethane. Collected organic phases were washed with water (2×50ml) and then treated with activated charcoal (2 g). After that the organic phase was evaporated to an oily residue (9.8g). 100 ml of acetone were added. The mixture was stirred at room temperature for at least 6 hours. The precipitated product was separated and washed with fresh acetone and dried at 65°C under reduced pressure for 3 hours.
Yield: 6.8g (88%)
Area % HPLC: Telmisartan 99.60%

### Example 4: Preparation of cyanotelmisartan

Into a reaction vessel 56 ml of acetonitrile, 0.95 g (14 mmol) of solid 85% KOH, 3.04 g (10 mmol) of 1,7'-dimethyl-2'-propyl-1H,3'H-2,5'-bibenzo[d]imidazole and 2.96 g (10.9 mmol) of 4'-(bromomethyl)-[1,1'-biphenyl]-2-carbonitrile were added. The reaction mixture was stirred at room temperature for 1 hour. The mixture was cooled to 0°C and filtered. The obtained product was washed with water and dried.
Yield: 4.18 g (85%)
HPLC: 96.5%

### Example 5: Preparation of cyanotelmisartan

Into a reaction vessel 115g (0.43mol) of methyl 4-methyl-2-propyl-1H-benzo[d]imidazole-6-carboxylate hydrochloride, 91g (0.86mol) of Na₂CO₃, 0.64g (0.0043ml) of NaI and 690 ml acetone were added. After the mixture was stirred for 2-3 hours, 116g (0.43mol) of 4'-(bromomethyl)biphenyl-2-carbonitrile were added. Then, the reaction mixture was heated to reflux for 15 hours, cooled to 10-15°C, stirred for one additional hour and filtered. The obtained solid was dried at 60°C for 24 hours. The obtained product methyl 1-((2'-cyanobiphenyl-4-yl)methyl)-4-methyl-2-propyl-1H-benzo[d]imidazole-6-carboxylate was isolated (172g, yield 95%).

Into a reaction vessel 172g (0.41mol) of methyl 1-((2'-cyanobiphenyl-4-yl)methyl)-4-methyl-2-propyl-1H-benzo[d]imidazole-6-carboxylate, 19.6g (0.49ml) of NaOH, 8.9g (0.49ml) of H₂O and 172ml of methanol were added. Then, the reaction mixture was heated to reflux for 2 hours and cooled to room temperature. Then, 4mol/L of HCl is added until pH is between 6 and 7, the mixture was filtered and the wet product was dried at 60°C for 24 hours. The obtained product 1-((2'-cyanobiphenyl-4-yl)methyl)-4-methyl-2-propyl-1H-benzo[d]imidazole-6-carboxylic acid was isolated (159g, yield 97%).

Into a reaction vessel 159g (0.375mol) of 1-((2'-cyanobiphenyl-4-yl)methyl)-4-methyl-2-propyl-1H-benzo[d]imidazole-6-carboxylic acid, 0.77 ml of dimethylformamide and 636ml of CH Cl were added. Then, 88.5g (0.75mol) of SOCl₂ were added at 0°C. The reaction mixture was heated to reflux for 7 to 8 hours and concentrated to dryness. Then, 318ml of ethyl acetate were added, the reaction mixture was filtrated and the wet product was used directly in the next step.

Into a reaction vessel 179g (1.69ml) of Na₂CO₃, 40g of NaCl, 636ml of H₂O and 636ml of ethyl acetate were added. The solution was cooled to 0-5°C and 219g (1.13mol) of N-methylbenzene-1,2-diamine dihydrochloride were added. The reaction mixture was stirred for 30min, cooled to -15 to -20°C and then the product obtained in the previous step was added. After stirring for 30min the temperature slowly rose to 0-5°C and after filtration the obtained product was dried at 60°C for 24 hours and isolated (173g, yield 90%).

Into a reaction vessel 173g (0.317mol) of the product obtained in the previous step, 0.97g (0.015ml) of H₃BO₃ and 1304ml of toluene were added. The reaction mixture was heated to reflux for 3-5hours and then cooled to 10-15°C and stirred for 2 hours. After filtration the wet product was dried at 60°C for 24 hours and cyanotelmisartan was isolated (143g, yield 86%).

100g of the crude product and 200ml of ethyl acetate were heated to reflux for 1hour, then cooled to room temperature and stirred for 1hour. After filtration the wet product was dried at 60°C for 24 hours and then isolated (95g, yield 95%).

### Example 6: Preparation of cyanotelmisartan

10g of methyl 4-methyl-2-propyl-1H-benzo[d]imidazole-6-carboxylate hydrochloride and 12.8g of K₂CO₃ were added to 40g of dimethylformamide. After the mixture was stirred for 1 hour at ambient temperature, 11.6g 4'-(bromomethyl)biphenyl-2-carbonitrile were added. The mixture was stirred for 6 to 7 hours at ambient temperature. Then, 200ml water were added drop-wise at temperatures around 30°C. The obtained mixture was filtered and washed with water. The cake was morcellated with 30ml of acetone at reflux temperature for 30 minutes, cooled to ambient temperature and continued to stir for 1 hour, filtrated and dried at 50°C for 4hours. The obtained product methyl 1-((2'-cyanobiphenyl-4-yl)methyl)-4-methyl-2-propyl-1H-benzo[d]imidazole-6-carboxylate was isolated as a white solid (14g, yield 90%).

Into a reaction vessel 172g of methyl 1-((2'-cyanobiphenyl-4-yl)methyl)-4-methyl-2-propyl-1H-benzo[d]imidazole-6-carboxylate, 19.6g of NaOH, 8.9g of H₂O and 138g of methanol were added. Then, the reaction mixture was heated to reflux for 4 hours and cooled to room temperature. Then, 344g of HCl (1.42M) were added until the pH was between 6 and 7. After filtration, the wet product was dried at 70°C under reduced pressure. The obtained product 1-((2'-cyanobiphenyl-4-yl)methyl)-4-methyl-2-propyl-1H-benzo[d]imidazole-6-carboxylic acid was isolated as white solid (159g, yield 97%).

Into a reaction vessel 159g of 1-((2'-cyanobiphenyl-4-yl)methyl)-4-methyl-2-propyl-1H-benzo[d]imidazole-6-carboxylic acid, 0.75g of dimethylformamide and 636g of CH₂Cl₂ are added. Then, at 0°C 88.5g of SOCl₂ were added. The reaction mixture was heated to reflux for 5 hours and concentrated to dryness. Then, 285ml of ethyl acetate was added, filtrated and the wet product was used directly in the next step.

Into a reaction vessel 179g of Na₂CO₃, 636g of H₂O and 570g of ethyl acetate were added. The solution was cooled to 0-5°C and 182g of N-methylbenzene-1,2-diamine dihydrochloride were added. The reaction mixture was stirred for 30min at a temperature of about 0 to 5°C, cooled to -15 to -20°C and then the product obtained in the previous step was added at a temperature below -10°C. After stirring for 30min the obtained mixture was filtered and the cake was washed with ethyl acetate. The cake was morcellated with 320g of CH₃OH at reflux temperature for 0.5 hours when 1200g of water is added. The mixture was cooled to ambient temperature for 0.5 hours with stirring and filtering. The residue was morcellated with 280g of ethyl acetate at a temperature of about 50°C for 1 hour and then filtered and dried at 60°C under reduced pressure. The product was grey to off-white solid (170g, yield 85%).

Into a reaction vessel 173g (0.317mol) of the product obtained in the previous step, 0.97g (0.015ml) of H₃BO₃ and 1304ml of toluene were added. The reaction mixture was heated to reflux for 3-5 hours and then cooled to 10-15°C and stirred for 2 hours. After filtration the wet product was dried at 60°C for 24 hours and cyanotelmisartan was isolated (143g, yield 86%). 100g of the crude product and 200ml of ethyl acetate were heated to reflux for 1 hour, then cooled to room temperature and stirred for 1hour. After filtration the wet product was dried at 60°C for 24 hours and then isolated (95g, yield 95%).

### Example 7: Bilayer tablet - Dry granulation

Bilayer tablet composition comprising 80 mg telmisartan and 12.5 mg HCTZ:

| **Telmisartan layer** | [mg] |
|---|---|
| Telmisartan | 80.0 |
| Meglumine | 24.0 |
| Sodium hydroxide | 6.7 |
| Povidone | 24.0 |
| Lactose monohydrate | 120.0 |
| Sorbitol | 299.7 |
| Magnesium stearate | 5.6 |
| **Total telmisartan layer** | **560.0** |
| | |

| **HCTZ layer** | [mg] |
|---|---|
| Hydrochlorothiazide | 12.5 |
| Mannitol | 187.5 |
| **Total HCTZ layer** | **200.0** |

Telmisartan, meglumine, sodium hydroxide and povidone were dissolved in water q.s. in order to prepare a granulation liquid. Lactose monohydrate was placed in a fluid-bed granulating machine and sprayed with the granulation liquid. After granulation had been completed, the granulate was dried and mixed with sorbitol and magnesium stearate to form a tablet layer composition ready for tableting.

Hydrochlorothiazide (HCTZ) was mixed with mannitol and compacted in a dry granulation apparatus. The obtained compact was milled into granules having an average particle size (volume mean diameter) of less than 30 µm. The particle size was determined by laser light scattering using a Malvern Mastersizer Apparatus MS 2000 equipped with a Hydro S dispersion unit. Vegetable oil was used as the dilution medium. Thus, a tablet layer composition containing only HCTZ and mannitol was obtained.

Bilayer tablets containing telmisartan and HCTZ were prepared by first introducing the telmisartan containing tablet layer composition into the tableting machine, followed by the HCTZ containing tablet layer composition. These two layers were then compressed on a rotary tablet press in a bilayer tableting mode.

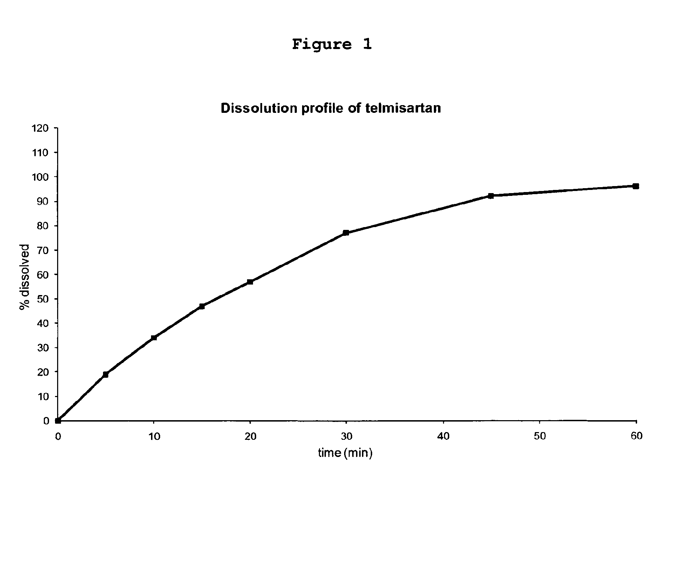

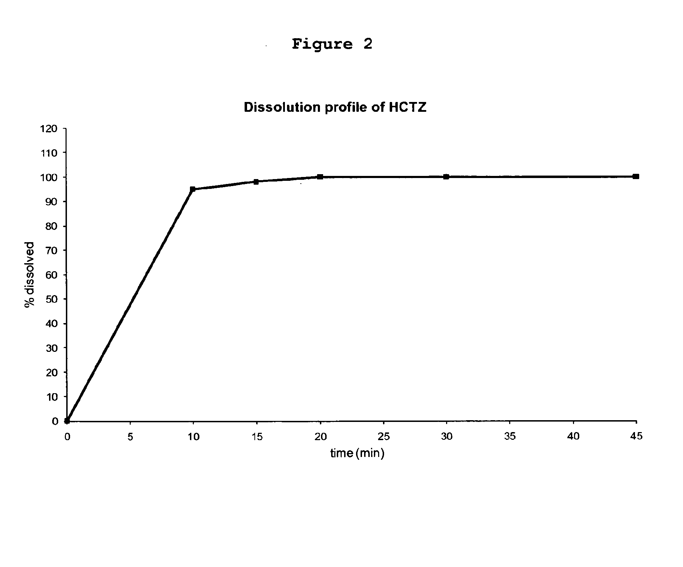

The dissolution profile of the finished bilayer tablet was determined in simulated gastric fluid (pH 2.0, USP apparatus II, 50 rpm). The dissolution profiles thus obtained are shown in Figure 1 (dissolution profile of telmisartan) and Figure 2 (dissolution profile of HCTZ). The dissolution profiles are comparable to that of commercially available Micardis Plus tablets.

### Example 8: Bilayer tablet - Fluid bed granulation

The telmisartan layer was the same as from Example 7.

| **HCTZ layer** | A [mg] | B [mg] | C [mg] | D [mg] |
|---|---|---|---|---|
| Hydrochlorothiazide | 25.00 | 25.00 | 25.00 | 25.00 |
| Mannitol | 25.00 | 119.55 | 119.55 | 25.00 |
| Hydroxypropyl cellulose | 1.00 | 2.25 | | |
| Povidone K30 | | | 2.25 | 1.00 |
| | | | | |
| Mannitol | 95.80 | | | 95.80 |
| Aerosil 200 | 0.20 | 0.20 | 0.20 | 0.20 |
| Magnesium stearat | 3.00 | 3.00 | 3.00 | 3.00 |
| Total HCTZ layer | 150.0 | 150.0 | 150.0 | 150.0 |

Hydrochlorothiazide and mannitol were granulated with hydroxypropyl cellulose or povidone dissolved in water. The obtained granulate was dried in a fluid bed dryer. The obtained granulate was then mixed with additional mannitol (if applicable), Aerosil 200 and magnesium stearate to form final composition ready for tableting.

Bilayer tablets containing telmisartan and HCTZ were prepared by first introducing the telmisartan containing mixture ready for tableting into the tableting machine, followed by introducing the HCTZ containing mixture ready for tableting. These two layers were then compressed on a rotary tablet press in a bilayer tableting mode.

### Example 9: Bilayer tablet - Direct compression

The telmisartan layer was the same as from Example 7.

| **HCTZ layer** | A [mg] | B [mg] | C [mg] | D [mg] |
|---|---|---|---|---|
| Hydrochlorothiazide | 25.00 | 25.00 | 25.00 | 25.00 |
| Xylitol | 166.60 | 50.00 | | |
| Lactose monohydrate | | | 50.00 | |
| Mannitol | | 116.60 | 116.60 | 166.60 |
| Aeroperl 300 | 0.40 | 0.40 | 0.40 | 0.40 |
| Sodium stearyl fumarate | 8.00 | 8.00 | 8.00 | 8.00 |
| **Total HCTZ layer** | 200.0 | 200.0 | 200.0 | 200.0 |

Hydrochlorothiazide was mixed with either xylitol, lactose monohydrate or mannitol as well as together with Aeroperl 300 and sodium stearyl fumarate to form a final composition ready for tableting by direct compression.

Bilayer tablets containing telmisartan and HCTZ were prepared by firstly introducing the telmisartan containing mixture ready for tableting into the tableting machine, followed by introducing the HCTZ containing mixture ready for tableting. These two layers were then compressed on a rotary tablet press in a bilayer tableting mode.

## Claims

1. A process for preparing telmisartan or a pharmaceutically acceptable salt thereof comprising
(a) providing a mixture comprising a derivative of telmisartan, an acid and a solvent, wherein the derivative of telmisartan is cyanotelmisartan or carboxamidotelmisartan and the acid is H₂SO₄,
(b) heating the obtained mixture,
(c) optionally converting the telmisartan to its pharmaceutically acceptable salt, and
(d) isolating telmisartan or its pharmaceutically acceptable salt.

2. Process according to claim 1, wherein the concentration of the derivative of telmisartan in the mixture of step (a) is more than 1 mol/L, preferably more than 1.5 mol/L, more preferably more than 1.8 mol/L such as 2 mol/L.

3. Process according to claim 1 or 2, wherein the concentration of the acid in the mixture of step (a) is more than 3 mol/L, preferably more than 6 mol/L.

4. Process according to any one of claims 1 to 3, wherein in step (a) the molar ratio between the acid and the derivative of telmisartan ranges from 2 to 12, more preferably from 3 to 8, and most preferably from 4 and 6.

5. Process according to any one of claims 1 to 4, wherein the solvent is selected from the group consisting of DMSO, *N*,*N*-dimethyformamide, *N*,*N*-dimethylacetamide, alcohols, water and mixtures thereof.

6. Process according to any one of claims 1 to 5, wherein the solvent comprises more than 90 vol.-%, preferably more than 93 vol.-%, more preferably more than 95 vol.-% and even more preferably more than 97 vol.-% of water.

7. Process according to claim 6, wherein the solvent is water.

8. Process according to any one of claims 1 to 7, wherein in step (b) the mixture is heated to a temperature between 80 and 150°C, preferably between 100 and 140°C, more preferably between 115 and 130°C.

9. Process for preparing telmisartan or a pharmaceutically acceptable salt thereof comprising
(a') providing a mixture comprising a derivative of telmisartan, an acid and a solvent, wherein the derivative of telmisartan is cyanotelmisartan or carboxamidotelmisartan and the acid is H₂SO₄,
(a") heating the obtained mixture,
(a''') cooling the obtained mixture,
(b) adjusting pH,
(c) extracting telmisartan with a first organic solvent,
(d) removing the first organic solvent,
(e) crystallizing telmisartan from a second organic solvent, and
(f) optionally converting the telmisartan to its pharmaceutically acceptable salts.

10. Process according to claim 9, wherein step (a') is performed according to step (a) of the process according to any one of claims 2 to 7.

11. Process according to claim 9 or 10, wherein step (a'') is performed according to step (b) of the process according to claim 8.

12. Process according to any one of claims 9 to 11, wherein in step (b) the pH is adjusted to 4 to 7, preferably to 4.5 to 6, more preferably to 5 to 5.5.

13. Process according to any one of claims 9 to 12, wherein the pH is adjusted by adding an aqueous solution of mineral bases, mineral acids, ammonia or organic bases, and preferably by adding an aqueous solution of NaOH or KOH.

14. Process according to any one of claims 9 to 13, wherein the first organic solvent is selected from the group consisting of an ester, an ether, toluene, a halogenated organic solvent and mixtures thereof, and preferably is dichloromethane.

15. Process according to any one of claims 9 to 14, wherein the second organic solvent is selected from the group consisting of an alcohol, an ester, a ketone an ether a nitrile and mixtures thereof, and preferably is acetone.

## Patentansprüche

1. Verfahren zur Herstellung von Telmisartan oder einem pharmazeutisch annehmbaren Salz davon, bei dem
(a) eine Mischung bereitgestellt wird, die ein Derivat von Telmisartan, eine Säure und ein Lösungsmittel enthält, wobei das Derivat von Telmisartan Cyanotelmisartan oder Carboxamidotelmisartan ist und die Säure H₂S0₄ ist,
(b) die erhaltene Mischung erwärmt wird,
(c) gegebenenfalls das Telmisartan in eines seiner pharmazeutisch annehmbaren Salze umgewandelt wird, und
(d) Telmisartan oder sein pharmazeutisch annehmbares Salz isoliert wird.

2. Verfahren nach Anspruch 1, bei dem die Konzentration des Derivats von Telmisartan in der Mischung in Schritt (a) größer als 1 mol/l, vorzugsweise größer als 1,5 mol/l, besonders bevorzugt größer als 1,8 mol/l, wie 2 mol/l, ist.

3. Verfahren nach Anspruch 1 oder 2, bei dem die Konzentration der Säure in der Mischung in Schritt (a) größer als 3 mol/l, vorzugsweise größer als 6 mol/l ist.

4. Verfahren nach einem der Ansprüche 1 bis 3, bei dem in Schritt (a) das molare Verhältnis zwischen der Säure und dem Derivat von Telmisartan im Bereich von 2 bis 12, besonders bevorzugt von 3 bis 8 und am meisten bevorzugt von 4 bis 6 liegt.

5. Verfahren nach einem der Ansprüche 1 bis 4, bei dem das Lösungsmittel ausgewählt ist aus der Gruppe bestehend aus DMSO, N,N-Dimethylformamid, N,N-Dimethylacetamid, Alkoholen, Wasser und Mischungen davon.

6. Verfahren nach einem der Ansprüche 1 bis 5, bei dem das Lösungsmittel mehr als 90 Vol.-%, vorzugsweise mehr als 93 Vol.-%, besonders bevorzugt mehr als 95 Vol.-% und noch bevorzugter mehr als 97 Vol.-% Wasser enthält.

7. Verfahren nach Anspruch 6, bei dem das Lösungsmittel Wasser ist.

8. Verfahren nach einem der Ansprüche 1 bis 7, bei dem in Schritt (b) die Mischung auf eine Temperatur zwischen 80 und 150°C, vorzugsweise zwischen 100 und 140°C, besonders bevorzugt zwischen 115 und 130°C erwärmt wird.

9. Verfahren zur Herstellung von Telmisartan oder einem pharmazeutisch annehmbaren Salz davon, bei dem
(a') eine Mischung bereitgestellt wird, die ein Derivat von Telmisartan, eine Säure und ein Lösungsmittel enthält, wobei das Derivat von Telmisartan Cyanotelmisartan oder Carboxamidotelmisartan ist und die Säure H₂SO₄ ist,
(a'') die erhaltene Mischung erwärmt wird,
(a''') die erhaltene Mischung gekühlt wird,
(b) der pH-Wert eingestellt wird,
(c) Telmisartan mit einem ersten organischen Lösungsmittel extrahiert wird,
(d) das erste organische Lösungsmittel entfernt wird,
(e) Telmisartan aus einem zweiten organischen Lösungsmittel kristallisiert wird, und
(f) gegebenenfalls das Telmisartan zu einem seiner pharmazeutisch annehmbaren Salze umgewandelt wird.

10. Verfahren nach Anspruch 9, bei dem Schritt (a') gemäß Schritt (a) des Verfahrens gemäß einem der Ansprüche 2 bis 7 durchgeführt wird.

11. Verfahren nach Anspruch 9 oder 10, bei dem Schritt (a'') gemäß Schritt (b) des Verfahrens gemäß Anspruch 8 durchgeführt wird.

12. Verfahren nach einem der Ansprüche 9 bis 11, bei dem in Schritt (b) der pH auf 4 bis 7, vorzugsweise 4,5 bis 6, besonders bevorzugt 5 bis 5,5 eingestellt wird.

13. Verfahren nach einem der Ansprüche 9 bis 12, bei dem der pH durch Zugabe einer wässrigen Lösung von Mineralbasen, Mineralsäuren, Ammoniak oder organischen Basen eingestellt wird und vorzugsweise durch Zugabe einer wässrigen Lösung von NaOH oder KOH eingestellt wird.

14. Verfahren nach einem der Ansprüche 9 bis 13, bei dem das erste organische Lösungsmittel ausgewählt ist aus der Gruppe bestehend aus einem Ester, einem Ether, Toluol, einem halogenierten organischen Lösungsmittel und Mischungen davon und vorzugsweise Dichlormethan ist.

15. Verfahren nach einem der Ansprüche 9 bis 14, bei dem das zweite organische Lösungsmittel ausgewählt ist aus der Gruppe bestehend aus einem Alkohol, einem Ester, einem Keton, einem Ether, einem Nitril und Mischungen davon und vorzugsweise Aceton ist.

## Revendications

1. Procédé de préparation de telmisartan ou d'un sel pharmacologiquement admissible de telmisartan, lequel procédé comporte les étapes suivantes :
a) préparer un mélange comprenant un dérivé de telmisartan, un acide et un solvant, dans lequel le dérivé de telmisartan est du nitrile de telmisartan ou du carboxamide de telmisartan et l'acide est de l'acide sulfurique,
b) faire chauffer le mélange obtenu,
c) en option, convertir le telmisartan en son sel pharmacologiquement admissible,
d) et isoler le telmisartan ou son sel pharmacologiquement admissible.

2. Procédé conforme à la revendication 1, dans lequel la concentration du dérivé de telmisartan dans le mélange de l'étape (a) vaut plus de 1 mol/L, de préférence plus de 1,5 mol/L et mieux encore plus de 1,8 mol/L, comme 2 mol/L.

3. Procédé conforme à la revendication 1 ou 2, dans lequel la concentration de l'acide dans le mélange de l'étape (a) vaut plus de 3 mol/L et de préférence plus de 6 mol/L.

4. Procédé conforme à l'une des revendications 1 à 3, dans lequel, dans l'étape (a), le rapport molaire entre l'acide et le dérivé de telmisartan vaut de 2 à 12, de préférence de 3 à 8, et mieux encore de 4 à 6.

5. Procédé conforme à l'une des revendications 1 à 4, pour lequel le solvant est choisi dans l'ensemble constitué par le diméthylsulfoxyde (DMSO), le N,N-diméthyl-formamide, le N,N-diméthylacétamide, les alcools et l'eau, ainsi que leurs mélanges.

6. Procédé conforme à l'une des revendications 1 à 5, dans lequel le solvant comprend de l'eau en une proportion de plus de 90 % en volume, de préférence de plus de 93 % en volume, mieux encore de plus de 95 % en volume, et surtout de plus de 97 % en volume.

7. Procédé conforme à la revendication 6, dans lequel le solvant est de l'eau.

8. Procédé conforme à l'une des revendications 1 à 7, dans lequel, dans l'étape (b), on chauffe le mélange à une température située entre 80 et 150 °C, de préférence entre 100 et 140 °C, et mieux encore entre 115 et 130 °C.

9. Procédé de préparation de telmisartan ou d'un sel pharmacologiquement admissible de telmisartan, lequel procédé comporte les étapes suivantes :
a') préparer un mélange comprenant un dérivé de telmisartan, un acide et un solvant, dans lequel le dérivé de telmisartan est du nitrile de telmisartan ou du carboxamide de telmisartan et l'acide est de l'acide sulfurique,
a") faire chauffer le mélange obtenu,
a"') faire refroidir le mélange obtenu,
b) ajuster le pH du mélange,
c) extraire le telmisartan à l'aide d'un premier solvant organique,
d) chasser ce premier solvant organique ;
e) faire cristalliser le telmisartan dans un deuxième solvant organique,
d) et en option, convertir le telmisartan en son sel pharmacologiquement admissible.

10. Procédé conforme à la revendication 9, dans lequel on réalise l'étape (a') conformément à l'étape (a) d'un procédé conforme à l'une des revendications 2 à 7.

11. Procédé conforme à la revendication 9 ou 10, dans lequel on réalise l'étape (a") conformément à l'étape (b) d'un procédé conforme à la revendication 8.

12. Procédé conforme à l'une des revendications 9 à 11, dans lequel, dans l'étape (b), on ajuste le pH à une valeur de 4 à 7, de préférence de 4,5 à 6, et mieux encore de 5 à 5,5.

13. Procédé conforme à l'une des revendications 9 à 12, dans lequel on ajuste le pH en ajoutant une solution aqueuse d'une base minérale, d'un acide minéral, d'ammoniac ou d'une base organique, et de préférence, en ajoutant une solution aqueuse de soude (NaOH) ou de potasse (KOH).

14. Procédé conforme à l'une des revendications 9 à 13, pour lequel le premier solvant organique est choisi dans l'ensemble formé par un ester, un éther, le toluène, un solvant organique halogéné et les mélanges de tels solvants, et est de préférence du dichlorométhane.

15. Procédé conforme à l'une des revendications 9 à 14, pour lequel le deuxième solvant organique est choisi dans l'ensemble formé par un alcool, un ester, une cétone, un éther, un nitrile et les mélanges de tels solvants, et est de préférence de l'acétone.
